(19) **Europäisches Patentamt / European Patent Office / Office européen des brevets**

(11) **EP 3 548 529 B1**

(12) **FASCICULE DE BREVET EUROPEEN**

(45) Date de publication et mention de la délivrance du brevet:
**14.10.2020 Bulletin 2020/42**

(21) Numéro de dépôt: **17818456.0**

(22) Date de dépôt: **01.12.2017**

(51) Int Cl.:
**C08F 290/06** *(2006.01)*    **B01J 13/18** *(2006.01)*
**C08F 2/22** *(2006.01)*

(86) Numéro de dépôt international:
**PCT/EP2017/081227**

(87) Numéro de publication internationale:
**WO 2018/100179 (07.06.2018 Gazette 2018/23)**

(54) **PROCÉDÉ DE PRÉPARATION DE MICROCAPSULES DE TAILLE CONTRÔLÉE COMPRENANT UNE ÉTAPE DE PHOTOPOLYMÉRISATION**

VERFAHREN ZUR HERSTELLUNG VON MIKROKAPSELN MIT KONTROLLIERTER GRÖSSE MIT EINER FOTOPOLYMERISATIONSSTUFE

PROCESS FOR PREPARING MICROCAPSULES OF CONTROLLED SIZE COMPRISING A PHOTOPOLYMERIZATION STEP

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.12.2016 FR 1661787**

(43) Date de publication de la demande:
**09.10.2019 Bulletin 2019/41**

(73) Titulaire: **Calyxia**
**94380 Bonneuil-sur-Marne (FR)**

(72) Inventeurs:
- **WALTERS, Jamie**
  **94380 Bonneuil-sur-Marne (FR)**
- **DEMOULIN, Damien**
  **75014 Paris (FR)**

(74) Mandataire: **Lavoix**
**2, place d'Estienne d'Orves**
**75441 Paris Cedex 09 (FR)**

(56) Documents cités:
**FR-A1- 2 867 075    US-A1- 2002 160 109**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet un procédé de préparation de microcapsules de taille contrôlée comprenant une étape de photopolymérisation.

Dans de nombreuses industries, notamment l'industrie chimique, cosmétique, agrochimique, de la peinture ou des carburants et lubrifiants, il est important d'encapsuler et d'isoler un matériau actif du milieu environnant, afin de protéger l'actif contre l'hydrolyse, la dégradation thermique, l'oxydation ou encore d'autres procédés qui peuvent réduire la performance de l'actif. En outre, de nombreuses applications au sein de ces industries exigent que les capsules produites aient une plage de taille étroite typiquement dans la gamme du micromètre (notamment entre 0,1 $\mu$m et 20 $\mu$m) par exemple pour avoir un meilleur contrôle sur leur performance globale.

La problématique liée à l'isolement d'un actif du milieu environnant afin d'améliorer la performance des actifs est un domaine relativement nouveau pour un certain nombre d'industries. Dans la plupart des industries non biologiques, les pertes de performances associées à des facteurs tels que l'hydrolyse, la dégradation thermique, l'oxydation et la réactivité croisée sont résolues en augmentant la concentration de la matière active pour atteindre le niveau désiré de performance, ce qui augmente le coût et génère également d'autres problèmes associés au produit formé à partir de tels procédés.

Ces dernières années, un grand nombre de procédés d'encapsulation ont été développés et rapportés dans la littérature, y compris le séchage par atomisation, l'évaporation de solvant, la polymérisation interfaciale et l'extrusion centrifuge parmi beaucoup d'autres. Cependant, pour les procédés d'encapsulation à l'échelle industrielle, les techniques d'émulsion dominent. De tels procédés ont recours à une étape formant une émulsion d'une huile hydrophobe ou d'une phase cireuse, dispersée dans un milieu aqueux ou alternativement une phase aqueuse, dispersée dans une huile hydrophobe ou un milieu cireux. Les deux phases sont émulsifiées en utilisant soit un homogénéisateur, soit un récipient agité équipé de chicanes et stabilisé en utilisant des tensioactifs, des lipides ou des émulsifiants polymériques. En variante, une réaction à l'interface entre les deux phases peut être utilisée pour la formation d'une enveloppe de polymère.

Toutefois, ces systèmes produisent des émulsions et des capsules qui sont polydisperses ou de taille trop élevée (au-dessus de 20 $\mu$m).

En outre, ces systèmes requièrent l'utilisation d'eau pour former l'une des phases. Ils requièrent également l'utilisation de tensioactifs ou émulsifiants similaires pour stabiliser l'émulsion, qui présentent l'inconvénient de pouvoir réagir avec l'encapsulant ou fournir des contaminants dans les différentes phases.

La présente invention a pour but de fournir des capsules contenant un actif par la mise en œuvre d'un procédé en masse afin de satisfaire les volumes pour répondre aux demandes des industries non biologiques.

La présente invention a également pour but de fournir un procédé d'encapsulation par double émulsion permettant d'obtenir des capsules de taille contrôlée, notamment de taille inférieure à 20 $\mu$m, voire 5 $\mu$m.

La présente invention a également pour but de fournir un procédé d'encapsulation d'actifs pouvant être mis en œuvre en l'absence d'eau et/ou de tensioactifs et d'émulsifiants.

Ainsi, la présente invention concerne un procédé de préparation de microcapsules solides comprenant les étapes suivantes :

> a) l'addition sous agitation d'une composition C1, comprenant au moins un actif, dans une composition polymérique photoréticulable C2, les compositions C1 et C2 n'étant pas miscibles l'une dans l'autre,
> ce par quoi on obtient une émulsion (E1) comprenant des gouttes de composition C1 dispersées dans la composition C2 ;
> b) l'addition sous agitation de l'émulsion (E1) dans une composition C3, les compositions C2 et C3 n'étant pas miscibles l'une dans l'autre,
> la viscosité de la composition C3 étant supérieure à la viscosité de l'émulsion (E1), et étant supérieure à 2 000 mPa.s à 25°C,
> ce par quoi on obtient une émulsion double (E2) comprenant des gouttes dispersées dans la composition C3 ;
> c) l'application d'un cisaillement à l'émulsion (E2), ladite vitesse de cisaillement appliquée étant inférieure à 1 000 s$^{-1}$,
> ce par quoi on obtient une émulsion double (E3) comprenant des gouttes de taille contrôlée dispersées dans la composition C3 ; et
> d) la photopolymérisation de la composition C2, ce par quoi on obtient des microcapsules solides dispersées dans la composition C3.

Le procédé de l'invention permet ainsi la production à l'échelle industrielle de populations de gouttes d'émulsion double de taille contrôlée et notamment inférieure à 20 $\mu$m. Le contrôle de la taille des capsules obtenues par le procédé de l'invention est dû en particulier au contrôle de la viscoélasticité des compositions C2 et C3.

Le procédé de l'invention permet la production de capsules de taille contrôlée par la mise en œuvre d'une étape de photopolymérisation, notamment de réticulation UV, de la phase intermédiaire de la double émulsion. Cette étape de photopolymérisation permet notamment de solidifier la couche intermédiaire des capsules et élimine ainsi toute coalescence.

De préférence, les microcapsules obtenues selon le procédé de l'invention ont un diamètre moyen (tel que mesuré par microscopie optique ou par MET ou par technique de diffusion de la lumière) compris entre 0,1 $\mu$m et 20 $\mu$m, et de préférence entre 1 $\mu$m et 20 $\mu$m.

### Etape a)

Pendant l'étape a), une composition C1 est ajoutée à une composition polymérique photoréticulable C2, cette étape étant effectuée sous agitation, ce qui signifie que la composition C2 est agitée, typiquement de façon mécanique, tandis que la composition C1 est ajoutée, et ce afin d'émulsifier le mélange des compositions C1 et C2.

L'addition de la composition C1 dans la composition C2 est typiquement effectuée goutte à goutte.

Pendant l'étape a), la composition C1 est à une température comprise entre 0°C et 100°C, de préférence entre 10°C et 80°C, et préférentiellement entre 15°C et 60°C. Pendant l'étape a), la composition C2 est à une température comprise entre 0°C et 100°C, de préférence entre 10°C et 80°C, et préférentiellement entre 15°C et 60°C.

Dans les conditions d'addition de l'étape a), les compositions C1 et C2 ne sont pas miscibles l'une dans l'autre, ce qui signifie que la quantité (en poids) de la composition C1 capable d'être solubilisée dans la composition C2 est inférieure ou égale à 5%, de préférence inférieure à 1%, et préférentiellement inférieure à 0,5%, par rapport au poids total de composition C2, et que la quantité (en poids) de la composition C2 capable d'être solubilisée dans la composition C1 est inférieure ou égale à 5%, de préférence inférieure à 1%, et préférentiellement inférieure à 0,5%, par rapport au poids total de composition C1.

Ainsi, lorsque la composition C1 entre en contact avec la composition C2 sous agitation, celle-ci est dispersée sous la forme de gouttes, dites gouttes simples.

L'immiscibilité entre les compositions C1 et C2 permet également d'éviter la migration de l'actif de la composition C1 vers la composition C2.

La composition C2 est agitée de manière à former une émulsion comprenant des gouttes de composition C1 dispersées dans la composition C2. Cette émulsion est aussi appelée « émulsion simple » ou émulsion C1-dans-C2.

Pour mettre en œuvre l'étape a), on peut utiliser tout type d'agitateur usuellement utilisé pour former des émulsions, comme par exemple un agitateur mécanique à pâles, un émulseur statique, un homogénéisateur à ultrasons, un homogénéisateur à membrane, un homogénéisateur à haute pression, un moulin colloïdal, un disperseur à haut pouvoir de cisaillement ou un homogénéisateur à haute vitesse.

### Composition C1

La composition C1 comprend au moins un actif A. Cette composition C1 sert de véhicule à l'actif A dans le procédé de l'invention, au sein des gouttes formées lors du procédé de l'invention et des capsules solides obtenues.

Selon une première variante du procédé de l'invention, la composition C1 est monophasique, c'est-à-dire qu'il s'agit de l'actif A pur ou bien d'une solution comprenant l'actif A sous forme solubilisée.

Selon un mode de réalisation, l'actif est solubilisé dans la composition C1.

Selon cette variante, la composition C1 consiste typiquement en une solution de l'actif A dans une solution aqueuse, ou un solvant organique, ou un mélange de solvants organiques, l'actif A étant présent selon une teneur massique comprise de 1% à 99%, par rapport à la masse totale de la composition C1. L'actif A peut être présent selon une teneur massique comprise de 5% à 95%, de 10% à 90%, de 20% à 80%, de 30% à 70%, ou de 40% à 60%, par rapport à la masse totale de la composition C1.

Selon un mode de réalisation, la composition C1 consiste en l'actif A.

Selon un autre mode de réalisation de l'invention, la composition C1 est une composition biphasique, ce qui signifie que l'actif est dispersé, soit sous forme liquide soit sous forme solide, dans la composition C1 et n'est pas totalement solubilisé dans ladite composition C1.

Selon un mode de réalisation, l'actif est dispersé sous la forme de particules solides dans la composition C1.

Selon ce mode de réalisation, la composition C1 peut consister en une dispersion de particules solides de l'actif dans un solvant organique ou dans un mélange de solvants organiques.

Selon ce mode de réalisation, la composition C1 peut consister en une dispersion de particules solides de l'actif dans une phase aqueuse, qui comprend de l'eau et éventuellement des solvants organiques hydrophiles.

L'actif utilisé est par exemple :

- un réticulant, un durcisseur, un catalyseur organique ou métallique (tel qu'un complexe organométallique ou inorganométallique de platine, de palladium, de titane, de molybdène, de cuivre, de zinc) utilisé pour polymériser des formulations de polymère, d'élastomère, de caoutchouc, de peinture, d'adhésif, de joint, de mortier, de vernis ou de revêtement ;

- un colorant ou un pigment destiné aux formulations d'élastomères, de peinture, de revêtement, d'adhésif, de joint, de mortier, ou de papier ;
- un parfum (au sens de la liste de molécule établie par l'International Fragrance Association (IFRA) et disponible sur le site internet www.ifraorg.org) destiné aux produits de détergence comme les lessives, aux produits de soin de la maison, aux produits cosmétiques et de soin de la personne, aux textiles, aux peintures, aux revêtements ;
- un arôme, une vitamine, un acide aminé, une protéine, un lipide, un probiotique, un antioxydant, un correcteur de pH, un préservateur pour les composés alimentaires et l'alimentation animale ;
- un adoucissant, un conditionnant pour les produits de détergence, les lessives, les cosmétiques et les produits de soin de la personne. A ce titre, les actifs utilisables sont par exemple énumérés dans les brevets US 6 335 315 et US 5 877 145 ;
- un agent anti altération de couleur (tel qu'un dérivé d'ammonium), un agent antimousse (tel qu'un éthoxylate d'alcool, un sulfonate d'alkylbenzène, un éthoxylate de polyéthylène, un alkyléthoxysulfate ou alkylsulfate) destiné aux produits de détergence et aux lessives et aux produits de soin de la maison ;
- un agent azurant, aussi appelé activateur de couleur (tel qu'un dérivé de stilbène, un dérivé de coumarine, un dérivé de pyrazoline, un dérivé de benzoxazole ou un dérivé de naphtalimide) destiné aux produits de détergence, aux lessives, aux cosmétiques et aux produits de soin de la personne ;
- un composé biologiquement actif tel qu'une enzyme, une vitamine, une protéine, un extrait végétal, un agent émollient, un agent désinfectant, un agent antibactérien, un agent anti-UV, un médicament destiné aux produits cosmétiques et de soin de la personne, aux textiles. Parmi ces composés biologiquement actifs on peut citer : les vitamines A, B, C, D et E, l'acide para aminobenzoïque, les acides alpha hydroxylés (comme l'acide glycolique, l'acide lactique, l'acide malique, l'acide tartrique ou l'acide citrique), le camphre, les céramides, les polyphénols (comme les flavonoïdes, l'acide phénolique, l'acide ellagique, le tocophérol, l'ubiquinol), l'hydroquinone, l'acide hyaluronique, l'isopropyl isostéarate, l'isopropyl palmitate, l'oxybenzone, le panthenol, la proline, le rétinol, le rétinyl palmitate, l'acide salicylique, l'acide sorbique, le sorbitol, le triclosan, la tyrosine ;
- un agent désinfectant, un agent antibactérien, un agent anti-UV, destiné aux peintures et revêtements ;
- un fertilisant, un herbicide, un insecticide, un pesticide, un fongicide, un repoussant ou un désinfectant destiné aux produits agrochimiques ;
- un agent ignifuge, aussi appelé retardateur de flamme, (tel qu'un polyol bromé comme le tetrabromobisphenol A, un composé organophosphoré halogéné ou non halogéné, un composé chloré, un trihydrate d'aluminium, un oxyde d'antimoine, un borate de zinc, un phosphore rouge, un mélamine, ou un dihydroxyde de magnésium) destiné aux matériaux plastiques, aux revêtement, aux peintures et aux textiles ;
- Un cristal photonique ou un photochromophore destiné aux peintures, aux revêtements et aux matériaux polymères formant les écrans incurvés et souples ;
- un produit connu par l'homme de l'art sous le nom de matériaux à changement de phase (PCM pour Phase Change Materials) capables d'absorber ou restituer de la chaleur lorsqu'ils subissent un changement de phase, destinés au stockage d'énergie. Des exemples de PCM et de leurs applications sont décrits dans "A review on phase change energy storage: materials and applications", Farid et al., Energy Conversion and Management, 2004, 45(9-10), 1597-1615. Comme exemples de PCM, on peut citer les sels fondus de phosphate d'aluminium, le carbonate d'ammonium, le chlorure d'ammonium, le carbonate de césium, le sulfate de césium, le citrate de calcium, le chlorure de calcium, l'hydroxyde de calcium, l'oxyde de calcium, le phosphate de calcium, le saccharate de calcium, le sulfate de calcium, le phosphate de cérium, le phosphate de fer, le carbonate de lithium, le sulfate de lithium, le chlorure de magnésium, le sulfate de magnésium, le chlorure de manganèse, le nitrate de manganèse, le sulfate de manganèse, l'acétate de potassium, le carbonate de potassium, le chlorure de potassium, le phosphate de potassium, le carbonate de rubidium, le sulfate de rubidium, le tétraborate de disodium, l'acétate de sodium, le bicarbonate de sodium, le bisulfate de sodium, le citrate de sodium, le chlorure de sodium, l'hydroxyde de sodium, le nitrate de sodium, le percarbonate de sodium, le persulfate de sodium, le phosphate de sodium, le propionate de sodium, le sélénite de sodium, le silicate de sodium, le sulfate de sodium, le tellurate de sodium, le thiosulfate de sodium, l'hydrophosphate de strontium, l'acétate de zinc, le chlorure de zinc, le thiosulfate de sodium, les cires hydrocarbonées paraffiniques, les polyéthylène glycols.

### Composition C2

La composition C2 est une composition photoréticulable ce qui signifie qu'il s'agit d'une composition capable de polymériser (réticuler) pour donner un matériau solide, pour former l'enveloppe polymérisée des microcapsules solides de l'invention.

Selon un mode de réalisation, la composition C2 est un liquide dont la viscosité à 25°C est comprise entre 500 mPa.s et 100 000 mPa.s.

La viscosité est mesurée au moyen d'un rhéomètre Haake Rheostress™ 600 équipé d'un cône de diamètre 60 mm et d'angle 2 degrés, et d'une cellule de régulation en température réglée à 25°C. La valeur de la viscosité est lue pour une vitesse de cisaillement égale à 10 s⁻¹.

De préférence, la viscosité de la composition C2 à 25°C est comprise entre 1 000 mPa.s et 50 000 mPa.s, préférentiellement entre 2 000 mPa.s et 25 000 mPa.s, et par exemple entre 3 000 mPa.s et 15 000 mPa.s.

De préférence, la viscosité de la composition C2 est supérieure à la viscosité de la composition C1.

Selon ce mode de réalisation, indépendamment de la viscosité de l'actif ou de ses propriétés chimiques, la cinétique de déstabilisation des gouttes de l'émulsion (E1) est significativement lente, ce qui permet à l'enveloppe des microcapsules d'être polymérisée pendant l'étape d) avant que l'émulsion ne se déstabilise. La polymérisation, une fois achevée, fournit alors une stabilisation thermodynamique.

Ainsi, la viscosité relativement élevée de la composition C2 assure la stabilité de l'émulsion (E1) obtenue à l'issue de l'étape a).

De préférence, la tension interfaciale entre les compositions C1 et C2 est faible. Typiquement, ces tensions interfaciales varient entre 0 mN/m et 50 mN/m, de préférence entre 0 mN/m et 20 mN/m.

La faible tension interfaciale entre les compositions C1 et C2 permet également de façon avantageuse d'assurer la stabilité de l'émulsion (E1) obtenue à l'issue de l'étape a).

Selon un mode de réalisation, le ratio entre le volume de composition C1 et le volume de composition C2 varie entre 1:10 et 10:1. De préférence, ce ratio est compris entre 1:3 et 5:1, préférentiellement entre 1:3 et 3:1.

Ce ratio peut être adapté afin de contrôler l'épaisseur de l'enveloppe des microcapsules polymérisées.

Selon un mode de réalisation, la composition C2 comprend au moins un monomère ou polymère, au moins un agent réticulant et au moins un photoinitiateur.

Selon un mode de réalisation, la composition C2 comprend de 50% à 99% en poids de monomère ou de polymère, ou un mélange de monomères ou polymères, par rapport au poids total de la composition C2.

Selon un mode de réalisation, la composition C2 comprend de 1% à 20% en poids d'agent réticulant ou d'un mélange d'agents réticulants, par rapport au poids total de la composition C2.

Selon un mode de réalisation, la composition C2 comprend de 0,1% à 5% en poids de photoinitiateur ou d'un mélange de photoinitiateurs, par rapport au poids total de la composition C2.

Selon un mode de réalisation, la composition C2 comprend de 0,001% à 70% en poids d'agent réticulant par rapport au poids de ladite composition C2.

Selon l'invention, le terme « monomère » ou « polymère » désigne toute unité de base adaptée pour la formation d'un matériau solide par polymérisation, soit seul soit en combinaison avec d'autres monomères ou polymères.

Ces monomères peuvent être choisis parmi les monomères comprenant au moins une fonction réactive choisie dans le groupe constitué des fonctions acrylate, méthacrylate, vinyl éther, N-vinyl éther, mercaptoester, thiolène, siloxane, époxy, oxétane, uréthane, isocyanate et peroxyde.

En particulier, les monomères peuvent être choisis parmi les monomères portant au moins une des fonctions réactives susmentionnées et portant en outre au moins une fonction choisie dans le groupe constitué des fonctions alkylamines primaires, secondaires et tertiaires, des fonctions amines quaternaires, des fonctions sulfate, sulfonate, phophate, phosphonate, carboxylate, hydroxyle, halogène, et leurs mélanges.

Les polymères utilisés dans la composition C2 peuvent être choisis parmi les polyéthers, polyesters, polyuréthanes, polyurées, polyéthylène glycols, polypropylène glycols, polyamides, polyacétals, polyimides, polyoléfines, polysulfures et les polydiméthylsiloxanes, lesdits polymères portant en outre au moins une fonction réactive choisie dans le groupe constitué des fonctions acrylate, méthacrylate, vinyl éther, N-vinyl éther, mercaptoester, thiolène, siloxane, époxy, oxétane, uréthane, isocyanate et peroxyde.

Parmi les exemples de tels polymères, on peut citer, mais de façon non limitative, les polymères suivants : poly(2-(1-naphthyloxy)-éthyl acrylate), poly(2-(2-naphthyloxy)-éthyl acrylate), poly(2-(2-naphthyloxy)-éthyl méthacrylate), polysorbitol diméthacrylate, polyacrylamide, poly((2-(1-naphthyloxy) éthanol), poly(2-(2-naphthyloxy) éthanol), poly(1-chloro-2,3-époxypropane), poly(n-butyl isocyanate), poly(N- vinyl carbazole), poly(N-vinyl pyrrolidone), poly(p-benzamide), poly(p-chlorostyrène), poly(p-méthyl styrène), poly(p-phénylène oxyde), poly(p-phénylène sulfure), poly(N-(méthacryloxyéthyl)succinimide), polybenzimidazol, polybutadiène, polybutylène téréphthalate, polychloral, polychloro trifluoro éthylène, polyéther imide, polyéther cétone, polyéther sulfone, polyhydridosilsesquioxane, poly(m-phénylène isophthalamide), poly(méthyl 2-acrylamido-2-méthoxyacéate), poly(2-acrylamido-2-méthylpropanesulfonique acide), poly-mono-butyl maléate, polybutylméthacrylate, poly(N-tert-butylméthacrylamide), poly(N-n-butylméthacrylamide), polycyclohexylméthacrylamide, poly(m-xylènebisacrylamide 2,3-diméthyl-1,3-butadiène,N,N-diméthylméthacrylamide), poly(n-butyl méthacrylate), poly(cyclohexyl méthacrylate), polyisobutyl méthacrylate, poly(4-cyclohexylstyrène), polycyclol acrylate, polycyclol méthacrylate, polydiéthyl éthoxyméthylènemalonate, poly(2,2,2-trifluoroéthyl méthacrylate), poly(1,1,1-triméthylolpropane triméthacrylate), polyméthacrylate, poly(N,N-diméthylaniline, dihydrazide), poly(dihydrazine isophthalique), polyacide isophthalique, polydiméthyl benzilketal, épichlorohydrine, poly(éthyl-3,3-diéthoxyacrylate), poly(éthyl-3,3-diméthylacrylate), poly(éthyl vinylcétone), poly(vinyl éthylcétone), poly(penten-3-one), polyformaldéhyde poly(diallyl acétal), polyfumaronitrile, polyglycéryl propoxy triacrylate, polyglycéryl triméthacrylate, polyglycidoxypropyltriméthoxysilane, polyglycidyl acrylate, poly(n-heptyl acrylate), poly(n-heptyl ester d'acide acrylique), poly(n-heptyl méthacrylate), poly(3-hydroxypropionitrile), poly(2-hydroxypropyl acrylate), poly(2-hydroxypropyl méthacrylate), poly(N-(méthacryloxyéthyl)phthalimide), poly(1,9-nonanediol diacrylate), poly(1,9-nonanediol diméthacrylate), poly(N-(n-propyl) acrylamide), poly(acide ortho-phtalique), poly(acide iso-phtalique), poly(acide 1,4-benzenedicarboxylique), poly(acide 1,3-benzenedicarboxylique), poly(acide phtalique), poly(mono-2-acryloxyéthyl ester), polyacide téréphthalique, polyanhydride phtalique, polyéthylène glycol diacrylate, polyéthylène glycol méthacrylate, polyéthylène glycol diméthacrylate, poly(isopropyl acrylate), polysorbitol pentaacrylate, polyvinyl bromoacétate, polychloroprène, poly(di-n-hexyl silylène), poly(di-n-propyl siloxane), polydiméthyl silylène, polydiphényl siloxane, polyvinyl propionate, polyvinyl triacétoxysilane, polyvinyl tris-tert-butoxysilane, polyvinyl butyral, polyalcool vinylique, polyacétate de vinyle, polyéthylène co-vinyl acétate, poly(bisphénol-A polysulfone), poly(1,3-dioxepane), poly(1,3-dioxolane), poly(1,4-phénylène vinylène), poly(2,6-diméthyl-1A-phénylène oxyde), poly(acide 4-hydroxybenzoique), poly(4-méthyl pentène-1), poly(4-vinyl pyridine), polyméthylacrylonitrile, polyméthylphénylsiloxane, polyméthylsilméthylène, polyméthylsilsesquioxane, poly(phénylsilsesquioxane), poly(pyromellitimide-1.4-diphényl éther), polytétrahydrofurane, polythiophène, poly(triméthylène oxyde), polyacrylonitrile, polyéther sulfone, polyéthylène-co-vinyl acétate, poly(perfluoréthylène propylène), poly(perfluoralkoxyl alcane), ou poly(styrène-acrylonitrile).

Par « agent réticulant », on entend un composé porteur d'au moins deux fonctions réactives susceptibles de réticuler un monomère ou un polymère, ou un mélange de monomères ou de polymères, lors de sa polymérisation.

L'agent réticulant peut être choisi parmi des molécules portant au moins deux fonctions choisies dans le groupe constitué des fonctions acrylate, méthacrylate, vinyl éther, N-vinyl éther, mercaptoester, thiolène, siloxane, époxy, oxétane, uréthane, isocyanate et peroxyde.

A titre d'agent réticulant, on peut notamment citer :

- les diacrylates, comme le 1,6-hexanediol diacrylate, le 1,6-hexanediol diméthacrylate, le polyéthylène glycol diméthacrylate, le 1,9-nonanediol diméthacrylate, le 1,4-butanediol diméthacrylate, le 2,2-bis(4-méthacryloxyphényl) propane, le 1,3-butanediol diméthacrylate, le 1,10-décanediol diméthacrylate, le bis(2-méthacryloxyéthyl) N,N'-1,9-nonylène biscarbamate, le 1,4-butanediol diacrylate, l'éthylène glycol diacrylate, le 1,5-pentanediol diméthacrylate, le 1,4-Phénylène diacrylate, l'allyl méthacrylate, le N,N'-méthylènebisacrylamide, le 2,2-bis[4-(2-hydroxy-3-méthacryloxypropoxy)phényl]propane, le tétraéthylène glycol diacrylate, l'éthylène glycol diméthacrylate, le diéthylène glycol diacrylate, le triéthylène glycol diacrylate, le triéthylène glycol diméthacrylate, le polyéthylène glycol diglycidyl éther, le N,N-diallylacrylamide, le 2,2-bis[4-(2-acryloxyéthoxy) phényl]propane, le glycidyl méthacrylate ;
- les acrylates multifonctionnels comme le dipentaérythritol pentaacrylate, le 1,1,1- triméthylolpropane triacrylate, le 1,1,1-triméthylolpropane triméthacrylate, l'éthylènediamine tétraméthacrylate, le pentaérythritol triacrylate, le pentaérythritol tétraacrylate ;
- les acrylates possédant également une autres fonction réactive, comme le propargyl méthacrylate, le 2-Cyanoéthyl acrylate, le tricyclodécane diméthanol diacrylate, l'hydroxypropyl méthacrylate, le N-acryloxysuccinimide, le N-(2-Hydroxypropyl)méthacrylamide, le N-(3-aminopropyl)méthacrylamide hydrochloride, le N-(t-BOC-aminopropyl)methacrylamide, le 2-aminoéthyl méthacrylate hydrochloride, le monoacryloxyéthyl phosphate, le o-nitrobenzyl méthacrylate, l'anhydride acrylique, le 2-(tert-butylamino)ethyl méthacrylate, le N,N-diallylacrylamide, le glycidyl méthacrylate, le 2-hydroxyéthyl acrylate, le 4-(2-acryloxyaéhoxy)-2-hydroxybenzophenone, le N-(Phthalimidométhyl)acrylamide, le cinnamyl méthacrylate.

Par « photoinitiateur », on entend un composé capable de se fragmenter sous l'effet d'un rayonnement lumineux.

Les photoinitiateurs utilisables selon la présente invention sont connus dans la technique et sont décrits, par exemple dans "Les photoinitiateurs dans la réticulation des revêtements", G. Li Bassi, Double Liaison - Chimie des Peintures, n°361, novembre 1985, p.34-41 ; "Applications industrielles de la polymérisation photoinduite", Henri Strub, L'Actualité Chimique, février 2000, p.5-13 ; et "Photopolymères : considérations théoriques et réaction de prise", Marc, J.M. Abadie, Double Liaison - Chimie des Peintures, n°435-436, 1992, p.28-34.

Ces photoinitiateurs englobent :

- les $\alpha$-hydroxycétones, comme la 2-hydroxy-2-méthyl-1-phényl-1-propanone, commercialisées par exemple sous les dénominations DAROCUR® 1173 et 4265, IRGACURE® 184, 2959, et 500 par la société BASF, et ADDITOL® CPK par la société CYTEC ;
- les $\alpha$-aminocétones, notamment la 2-benzyl-2-diméthylamino-1-(4-morpholinophényl)-butanone-1, commercialisées par exemple sous les dénominations IRGACURE® 907 et 369 par la société BASF ;
- les cétones aromatiques commercialisées par exemple sous la dénomination ESACURE® TZT par LAMBERTI ; ou encore les thioxanthones commercialisées par exemple sous la dénomination ESACURE® ITX par LAMBERTI, et les quinones. Ces cétones aromatiques nécessitent le plus souvent la présence d'un composé donneur d'hydrogène tel que les amines tertiaires et notamment les alcanolamines. On peut notamment citer l'amine tertiaire ESACURE® EDB commercialisée par la société LAMBERTI.

- les dérivés α-dicarbonyles dont le représentant le plus courant est le benzyldiméthylcétal commercialisé sous la dénomination IRGACURE® 651 par BASF. D'autres produits commerciaux sont commercialisés par la société LAMBERTI sous la dénomination ESACURE® KB1, et
- les oxydes d'acylphosphine, tels que par exemple les oxydes de bis-acylphosphine (BAPO) commercialisés par exemple sous les dénominations IRGACURE® 819, 1700, et 1800, DAROCUR® 4265, LUCIRIN® TPO, et LUCIRIN® TPO-L par la société BASF.

Parmi les photoinitiateurs, on peut également mentionner les cétones aromatiques comme la benzophénone, les phénylglyoxylates, comme l'ester méthylique de l'acide phényl glyoxylique, les esters d'oxime, comme le [1-(4-phénylsulfanylbenzoyl)heptylidèneamino]benzoate, les sels de sulfonium, les sels d'iodonium et les oxime sulfonates.

Selon un mode de réalisation, la composition C2 peut en outre comprendre un monomère ou un polymère additionnel capable d'améliorer les propriétés de l'enveloppe des microcapsules et/ou de donner de nouvelles propriétés à l'enveloppe des microcapsules.

Parmi ces monomères ou polymères additionnels, on peut citer les monomères ou polymères portant un groupe sensible au pH, à la température, aux UV ou aux IR.

Ces monomères ou polymères additionnels peuvent induire la rupture des microcapsules solides et par la suite la libération de leur contenu, après une stimulation via le pH, la température, les UV ou les IR.

Ces monomères ou polymères additionnels peuvent être choisis parmi les monomères ou polymères portant au moins une fonction réactive choisie dans le groupe constitué des fonctions acrylate, méthacrylate, vinyl éther, N-vinyl éther, mercaptoester, thiolène, siloxane, époxy, oxétane, uréthane, isocyanate et peroxyde, et portant également l'un des groupes suivants :

- un groupe hydrophobe tel qu'un groupe fluoré, par exemple le trifluoroéthyl méthacrylate, le trifluoroéthyl acrylate, le tétrafluoropropyl méthacrylate, le pentafluoropropyl acrylate, le hexafluorobutyl acrylate, ou le fluorophényl isocyanate ;
- un groupe sensible au pH comme les amines primaires, secondaires ou tertiaires, les acides carboxyliques, les groupes phosphate, sulfate, nitrate, ou carbonate ;
- un groupe sensible aux UV ou clivable par UV (ou groupe photochromique) comme les groupes azobenzène, spiropyrane, 2-diazo-1,2-naphthoquinone, o-nitrobenzylé, thiol, ou 6-nitro-veratroyloxycarbonyle, par exemple poly(éthylène oxyde)-bloc-poly(2-nitrobenzylméthacrylate), et d'autres copolymères à blocs, comme décrit notamment dans Liu et al., Polymer Chemistry 2013, 4, 3431-3443 ;
- un groupe sensible aux IR ou clivable par IR comme le o-nitrobenzyle ou le 2-diazo-1,2-naphthoquinone, par exemple les polymères décrits dans Liu et al., Polymer Chemistry 2013, 4, 3431-3443 ; et
- un groupe sensible à la température comme le poly(N-isopropylacrylamide).

*Etape b)*

Pendant l'étape b), l'émulsion (E1) obtenue à l'étape a) est ajoutée à une composition C3, cette étape étant effectuée sous agitation, ce qui signifie que la composition C3 est agitée, typiquement de façon mécanique, tandis que l'émulsion (E1) est ajoutée, et ce afin d'émulsifier le mélange des compositions C1, C2 et C3.

L'addition de l'émulsion (E1) dans la composition C3 est typiquement effectuée goutte à goutte.

Pendant l'étape b), l'émulsion (E1) est à une température comprise entre 15°C et 60°C. Pendant l'étape b), la composition C3 est à une température comprise entre 15°C et 60°C.

Dans les conditions d'addition de l'étape b), les compositions C2 et C3 ne sont pas miscibles l'une dans l'autre, ce qui signifie que la quantité (en poids) de la composition C2 capable d'être solubilisée dans la composition C3 est inférieure ou égale à 5%, de préférence inférieure à 1%, et préférentiellement inférieure à 0,5%, par rapport au poids total de composition C3, et que la quantité (en poids) de la composition C3 capable d'être solubilisée dans la composition C2 est inférieure ou égale à 5%, de préférence inférieure à 1%, et préférentiellement inférieure à 0,5%, par rapport au poids total de composition C2.

Ainsi, lorsque l'émulsion (E1) entre en contact avec la composition C3 sous agitation, celle-ci est dispersée sous la forme de gouttes, dites gouttes doubles, la dispersion de ces gouttes d'émulsion (E1) dans la phase continue C3 étant appelée émulsion (E2).

Typiquement, une goutte double formée pendant l'étape b) correspond à une goutte simple de composition C1 telle que décrite ci-dessus, entourée par une enveloppe de composition C2 qui encapsule totalement ladite goutte simple.

La goutte double formée pendant l'étape b) peut également comprendre au moins deux gouttes simples de composition C1, lesdites gouttes simples étant entourées par une enveloppe de composition C2 qui encapsule totalement lesdites gouttes simples.

Ainsi, lesdites gouttes doubles comprennent un cœur constitué d'une ou plusieurs gouttes simples de composition C1, et une couche de composition C2 entourant ledit cœur.

L'émulsion (E2) résultante est généralement une émulsion double polydisperse (émulsion C1-dans-C2-dans-C3 ou émulsion C1/C2/C3), ce qui signifie que les gouttes doubles n'ont pas une nette distribution de taille dans l'émulsion (E2).

L'immiscibilité entre les compositions C2 et C3 permet d'éviter le mélange entre la couche de composition C2 et la composition C3 et assure ainsi la stabilité de l'émulsion (E2).

L'immiscibilité entre les compositions C2 et C3 permet également d'empêcher l'actif de la composition C1 de migrer du cœur des gouttes vers la composition C3.

Pour mettre en œuvre l'étape b), on peut utiliser tout type d'agitateur usuellement utilisé pour former des émulsions, comme par exemple un agitateur mécanique à pâles, un émulseur statique, un homogénéisateur à ultrasons, un homogénéisateur à membrane, un homogénéisateur à haute pression, un moulin colloïdal, un disperseur à haut pouvoir de cisaillement ou un homogénéisateur à haute vitesse.

***Composition C3***

Selon l'invention, la viscosité de la composition C3 à 25°C est supérieure à la viscosité de l'émulsion (E1) à 25°C.

De préférence, la viscosité de la composition C3 à 25°C est comprise entre 3 000 mPa.s et 100 000 mPa.s, préférentiellement entre 5 000 mPa.s et 80 000 mPa.s, par exemple entre 7 000 mPa.s et 70 000 mPa.s.

Selon ce mode de réalisation, étant donné la viscosité très élevée de la phase continue formée par la composition C3, la vitesse de déstabilisation des gouttes doubles de l'émulsion (E2) est significativement lente par rapport à la durée du procédé de l'invention, ce qui fournit alors une stabilisation cinétique des émulsions (E2) puis (E3) jusqu'à ce que la polymérisation de l'enveloppe des capsules ne soit achevée. Les capsules une fois polymérisées sont stables thermodynamiquement.

Ainsi, la viscosité très élevée de la composition C3 assure la stabilité de l'émulsion (E2) obtenue à l'issue de l'étape b).

De préférence, la tension interfaciale entre les compositions C2 et C3 est faible. La faible tension interfaciale entre les compositions C2 et C3 permet également de façon avantageuse d'assurer la stabilité de l'émulsion (E2) obtenue à l'issue de l'étape b).

Selon un mode de réalisation, le ratio entre le volume d'émulsion (E1) et le volume de composition C3 varie entre 1:10 et 10:1. De préférence, ce ratio est compris entre 1:9 et 3:1, préférentiellement entre 1:9 et 1:1.

Ce ratio peut être adapté afin de contrôler la quantité totale de matériel actif encapsulé parmi la population résultante de microcapsules polymérisées.

Selon un mode de réalisation, la composition C3 comprend au moins un polymère branché, de préférence de poids moléculaire supérieur à 5 000 g.mol$^{-1}$, préférentiellement entre 10 000 g.mol$^{-1}$ et 500 000 g.mol$^{-1}$, par exemple entre 50 000 g.mol$^{-1}$ et 300 000 g.mol$^{-1}$.

Par « polymère branché » (ou polymère ramifié), on entend un polymère présentant au moins un point de ramification entre ses deux groupes terminaux, un point de ramification (aussi appelé point de branchement) étant un point d'une chaîne sur lequel est fixée une chaîne latérale aussi appelée branche ou chaîne pendante.

Parmi les polymères branchés, on peut par exemple citer les polymères greffés, en peigne ou encore les polymères en étoile ou les dendrimères.

Selon un mode de réalisation, la composition C3 comprend au moins un polymère de poids moléculaire supérieur à 5 000 g.mol$^{-1}$, préférentiellement entre 10 000 g.mol$^{-1}$ et 500 000 g.mol$^{-1}$, par exemple entre 50 000 g.mol$^{-1}$ et 300 000 g.mol$^{-1}$.

A titre de polymère utilisable dans la composition C3, on peut citer les composés suivants, utilisés seuls ou bien mélangés entre eux :

- les dérivés de cellulose, tels que les éthers de cellulose : le méthyl cellulose, l'éthyl cellulose, l'hydroxyéthyl cellulose, le méthylhydroxyéthyl cellulose, l'éthylhydroxyéthyl cellulose, le carboxyméthyl cellulose, l'hydroxypropyl cellulose ou le méthylhydroxypropyl cellulose ;
- les polyacrylates (encore appelés carbomères), tels que l'acide polyacrylique (PAA), l'acide polyméthacrylique (PMAA), le poly(hydroxyéthyl méthacrylate) (pHEMA), le poly(N-2-hydroxypropyl méthacrylate) (pHPMA) ;
- les polyacrylamides tels que le poly(N-isopropylacrylamide) (PNIPAM) ;
- le polyvinylpyrrolidone (PVP) et ses dérivés ;
- l'alcool polyvinylique (PVA) et ses dérivés ;
- le poly(éthylène glycol), le poly(propylène glycol) et leurs dérivés, tels que le poly(éthylène glycol) acrylate/méthacrylate, le poly(éthylène glycol) diacrylate/diméthacrylate, le polypropylène carbonate ;

- les polysaccharides tels que les carraghénanes, les gommes de caroube ou gommes tara, le dextran, les gommes xanthanes, le chitosane, l'agarose, les acides hyaluroniques, la gomme gellane, la gomme de guar, la gomme arabique, la gomme adragante, la gomme diutane, la gomme d'avoine, la gomme karaya, la gomme ghatti, la gomme curdlan, la pectine, la gomme konjac, l'amidon ;
- les dérivés protéinés tels que la gélatine, le collagène, la fibrine, la polylysine, l'albumine, la caséine ;
- les dérivés de silicone tels que le polydimethylsiloxane (aussi appelé diméthicone), les alkyl silicones, les aryl silicones, les alkyl aryl silicones, les polyéthylène glycol diméthicones, les polypropylène glycol diméthicone ;
- les cires, telles que les cires diester (diesters d'alcanediol, diesters d'hydroxylacides), les cires triester (triacylglycérols, triesters d'alcane-1,2-diol, de ω-hydroxy acide et d'acide gras, esters d'acide hydroxymalonique, d'acide gras et d'alcool, triesters d'hydroxylacides, d'acide gras et d'alcool gras, triesters d'acide gras, d'hydroxylacide et de diol) et les cires polyesters (polyesters d'acides gras). Les esters d'acides gras utilisables à titre de cires dans le cadre de l'invention sont par exemple le palmitate de cétyle, l'octanoate de cétyle, le laurate de cétyle, le lactate de cétyle, l'isononanoate de cétyle, le stéarate de cétyle, le stéarate de stéaryle, le stéarate de myristyle, le myristate de cétyle, le stéarate d'isocétyle, le trimyristate de glycéryle, le tripalmitate de glycéryle, le monostéarate de glycéryle ou le palmitate de glycéryle et de cétyle ;
- les acides gras utilisables comme cires tels que l'acide cérotique, l'acide palmitique, l'acide stéarique, l'acide dihydroxystéarique, l'acide béhénique, l'acide lignocérique, l'acide arachidique, l'acide myristique, l'acide laurique, l'acide tridécyclique, l'acide pentadécyclique, l'acide margarique, l'acide nonadécyclique, l'acide hénéicosylique, l'acide tricosylique, l'acide pentacosylique, l'acide heptacosylique, l'acide montanique ou l'acide nonacosylique ;
- les sels d'acide gras notamment les sels d'aluminium d'acide gras tels que l'aluminium stéarate, l'hydroxyl aluminium bis(2-éthylhexanoate) ;
- l'huile de jojoba isomérisée ;
- l'huile de tournesol hydrogénée ;
- l'huile de coprah hydrogénée ;
- l'huile de lanoline hydrogénée ;
- l'huile de ricin et ses dérivés, notamment l'huile de ricin hydrogénée modifiée ou les composés obtenus par estérification d'huile de ricin avec des alcools gras ;
- les polyuréthanes et leurs dérivés ;
- les polymères styréniques tels que le styrène butadiène ;
- les polyoléfines telles que le polyisobutène.

Selon un mode de réalisation, la composition C3 comprend des particules solides telles que des argiles, des silices et des silicates.

A titre de particules solides utilisables dans la composition C3, on peut citer les argiles et silicates appartenant notamment à la catégorie des phyllosilicates (encore appelées silices en feuillets). A titre d'exemple de silicate utilisable dans le cadre de l'invention, on peut citer la Bentonite, l'Hectorite, l'Attapulgite, la Sepiolite, la Montmorillonite, la Saponite, la Sauconite, la Nontronite, la Kaolinite, le Talc, la Sepiolite, la Craie. Les silices synthétiques pyrogénées peuvent également être utilisées. Les argiles, silicates et silices citées précédemment peuvent avantageusement être modifiées par des molécules organiques telles que des polyéthers, des amides éthoxylées, des sels d'ammonium quaternaires, des diamines à longue chaîne, des esters à longue chaîne, des polyéthylène glycols, des polypropylène glycols.

Ces particules peuvent être utilisées seules ou mélangées entre elles.

Selon un mode de réalisation, la composition C3 comprend au moins un polymère de poids moléculaire supérieur à 5 000 g.mol$^{-1}$ et des particules solides. Tout mélange des composés cités précédemment peut être utilisé.

***Etape c)***

Dans l'étape c), l'émulsion (E2), constituée de gouttes polydisperses dispersées dans une phase continue, est soumise à un cisaillement, par exemple dans un mélangeur, à une faible vitesse de cisaillement, à savoir inférieure à 1 000 s$^{-1}$.

Selon un mode de réalisation, la vitesse de cisaillement appliquée à l'étape c) est comprise entre 10 s$^{-1}$ et 1 000 s$^{-1}$.

De préférence, la vitesse de cisaillement appliquée à l'étape c) est strictement inférieure à 1 000 s$^{-1}$.

Pendant l'étape c), l'émulsion (E2) est introduite dans un mélangeur et est ensuite soumise à un cisaillement qui résulte en la formation d'une troisième émulsion, l'émulsion (E3). Cette émulsion (E3) est chimiquement identique à l'émulsion (E2) mais elle est constituée de gouttes doubles monodisperses, et non polydisperses comme (E2).

Typiquement, l'émulsion (E3) consiste en une dispersion de gouttes doubles comprenant un cœur constitué d'une ou plusieurs gouttes simples de composition C1, et une couche de composition C2 entourant ledit cœur, lesdites gouttes doubles étant dispersées dans la composition C3.

La différence entre l'émulsion (E2) et l'émulsion (E3) est la variation de taille des gouttes doubles : les gouttes de l'émulsion (E2) sont polydisperses en taille alors que les gouttes de l'émulsion (E3) sont monodisperses grâce au mécanisme de fragmentation ayant lieu durant l'étape c).

Les gouttes d'émulsion (E2) ne peuvent être fragmentées efficacement en des gouttes fines et monodisperses d'émulsion (E3) que si une contrainte de cisaillement élevée leur est appliquée.

La contrainte de cisaillement σ appliquée à une goutte d'émulsion (E2) est définie comme la force tangentielle par unité de surface de goutte résultant du cisaillement macroscopique appliqué à l'émulsion lors de son agitation au cours de l'étape c).

La contrainte de cisaillement σ (exprimée en Pa), la viscosité de la composition C3 η (exprimée en Pa s) et la vitesse de cisaillement γ (exprimée en s$^{-1}$) appliquée à l'émulsion (E2) lors de son agitation au cours de l'étape c) sont reliées par l'équation suivante :

$$\sigma = \eta\gamma$$

Ainsi, la viscosité élevée de la composition C3 permet d'appliquer une très haute contrainte de cisaillement aux gouttes d'émulsion (E2) dans le mélangeur, même si la vitesse de cisaillement est faible et le cisaillement inhomogène.

Pour mettre en œuvre l'étape c), on peut utiliser tout type d'agitateur usuellement utilisé pour former des émulsions, comme par exemple un agitateur mécanique à pâles, un émulseur statique, un homogénéisateur à ultrasons, un homogénéisateur à membrane, un homogénéisateur à haute pression, un moulin colloïdal, un disperseur à haut pouvoir de cisaillement ou un homogénéisateur à haute vitesse.

Selon un mode de réalisation préféré, on utilise un émulseur simple tel qu'un agitateur mécanique à pâles ou un émulseur statique pour mettre en œuvre l'étape a). En effet, ceci est possible car le procédé de l'invention ne requiert ni un cisaillement contrôlé ni un cisaillement plus grand que 1 000 s$^{-1}$.

### Etape d)

L'étape d) consiste à soumettre l'émulsion (E3) à une photopolymérisation, ce qui va permettre la photopolymérisation de la composition C2.

Cette étape va permettre d'obtenir des microcapsules encapsulant l'actif tel que défini ci-dessus.

Selon un mode de réalisation, l'étape d) consiste à exposer l'émulsion (E3) à une source de lumière apte à initier la photopolymérisation de la composition C2.

De préférence, la source de lumière est une source de lumière UV.

Selon un mode de réalisation, la source de lumière UV émet dans la gamme de longueur d'onde comprise entre 100 nm et 400 nm.

Selon un mode de réalisation, l'émulsion (E3) est exposée à une source de lumière pendant une durée inférieure à 15 minutes, et de préférence pendant 5 à 10 minutes.

Pendant l'étape d), l'enveloppe des gouttes doubles susmentionnées, constituée de composition C2 photoréticulable, est réticulée et ainsi convertie en une enveloppe polymérique viscoélastique, encapsulant et protégeant l'actif de sa libération en l'absence d'un déclenchement mécanique.

La composition obtenue à l'issue de l'étape d), comprenant des microcapsules solides dispersées dans la composition C3, est prête à l'emploi et peut être utilisée sans qu'aucune étape supplémentaire de post-traitement des capsules ne soit requise.

L'épaisseur de l'enveloppe des microcapsules ainsi obtenues est typiquement comprise entre 10 nm et 2,5 μm, de préférence entre 100 nm et 1 000 nm.

Selon un mode de réalisation, les microcapsules solides obtenues à l'issue de l'étape d) sont dépourvues d'eau et/ou de tensioactif.

Le procédé de l'invention présente l'avantage de ne pas nécessiter d'eau, dans aucune des étapes décrites. Le procédé de l'invention permet ainsi d'encapsuler des composés sensibles à l'eau.

Le procédé de l'invention présente l'avantage de ne pas nécessiter de tensioactif, dans aucune des étapes décrites. Le procédé de l'invention permet ainsi de réduire la présence d'additifs qui pourraient modifier les propriétés du produit final obtenu après libération de l'actif.

## EXEMPLES

### Exemple 1 : Préparation de microcapsules solides grâce à une phase C3 très visqueuse et un cisaillement faible

Cet exemple démontre l'utilisation d'une composition C3 visqueuse permettant d'obtenir des capsules monodisperses de taille inférieure à 20 μm même en appliquant un cisaillement très faible à la double émulsion (E2).

*Composition de C1, C2 et C3 :*

- La composition C1 est une solution d'alginate (actif) à 5% en masse.
- La composition C2 est un mélange de 69% en poids de polymère CN981 (oligomère polyacrylate de la marque Sartomer, Arkema) ; 30% en poids d'hexanediol diacrylate (agent réticualnt) et de 1% en poids de Darocure 1173 (photoinitiateur).
- La composition C3 est une solution d'alginate à 15% en masse, de viscosité 63 000 s$^{-1}$ à 25°C.

*Fabrication des microcapsules :*

Un agitateur mécanique (Heidolph RZR 2021) équipé d'une hélice d'agitation de type défloculeuse de diamètre 3 cm est utilisé pour réaliser toutes les étapes d'émulsification.

Etape a) : la composition C1 est ajoutée goutte à goutte à la composition C2 à un ratio C1:C2 = 30:70 en poids sous agitation à 500 tpm.

Etape b) : l'émulsion (E1) obtenue à l'étape précédente est ajoutée goutte à goutte à la composition C3 à un ratio E1:C3 = 10:90 en poids sous agitation à 500 tpm.

Etape c) : l'émulsion (E2) ainsi obtenue est laissée sous agitation à 500 tpm pendant 10 minutes. Le cisaillement appliqué par une hélice d'agitation est très peu contrôlé. Dans les conditions de l'étape c) le cisaillement appliqué à l'émulsion (E2) peut être estimé à moins de 500 s$^{-1}$ (pour le détail du calcul, on se référera à : Metzner AB, Otto RE. Agitation of non-Newtonian fluids. AIChE J (1957) 3: 3-10 ; Wu, J et al., Estimation of agitator flow shear rate. AIChE J (2006) 52: 2323-2332).

Etape d) : l'émulsion monodisperse (E3) ainsi obtenue est irradiée pendant 10 minutes à l'aide d'une source de lumière UV (Dymax LightBox ECE 2000) ayant une intensité lumineuse maximale de 0,1 W/cm$^2$ à une longueur d'onde de 365 nm, pour permettre la réticulation des capsules.

La distribution de taille des capsules ainsi obtenues est mesurée par technique de diffusion de la lumière à l'aide d'un Mastersizer 3000 (Malvern Instruments) équipé d'une cellule de meure Hydro SV. La taille moyenne des capsules est mesurée à 26 μm. La largeur à mi-hauteur de la distribution de taille (considérée comme un moyen simple d'évaluer la monodispersité des capsules) est mesurée à 31 μm.

### Exemple 2 : Préparation de microcapsules solides grâce à une phase C3 très visqueuse et un cisaillement élevé

Cet exemple démontre qu'avec la même formulation que dans l'exemple 1, l'application d'un cisaillement élevé n'améliore pas la monodispersité des capsules obtenues.

Dans cet exemple, une émulsion (E2) identique en tous points à celle de l'exemple 1 est tout d'abord obtenue. Celle-ci est alors séparée en 2 fractions de volume égal qui sont introduites dans une cellule à haut cisaillement de type Couette fabriquée par l'entreprise TSR33. Cette cellule est composée de 2 cylindres concentriques, l'un mobile et l'autre fixe, séparés par un entrefer de 100 μm. La rotation du cylindre mobile permet d'appliquer un cisaillement uniforme à toute l'émulsion contenue dans l'entrefer. La première fraction de l'émulsion (E2) est soumise à un cisaillement de 6 300 s$^{-1}$ et la seconde fraction à un cisaillement de 14 300 s$^{-1}$.

Les émulsions (E3) ainsi obtenues sont irradiées pendant 10 minutes à l'aide d'une source de lumière UV (Dymax LightBox ECE 2000) ayant une intensité lumineuse maximale de 0,1 W/cm$^2$ à une longueur d'onde de 365 nm, pour permettre la réticulation des capsules.

La distribution de taille des capsules ainsi obtenues est mesurée par technique de diffusion de la lumière à l'aide d'un Mastersizer 3000 (Malvern Instruments) équipée d'une cellule de meure Hydro SV. La taille moyenne des capsules et la largeur à mi-hauteur de la distribution de taille obtenues sont récapitulées dans le tableau ci-dessous et comparées aux valeur obtenues dans l'Exemple 1. On voit clairement que l'application d'un haut cisaillement ne diminue pas la taille moyenne et la monodispersité des capsules.

| | Exemple 1 | Exemple 2 | |
|---|---|---|---|
| Cisaillement | < 500 s$^{-1}$ (pâle défloculeuse) | 6 300 s$^{-1}$ (cellule de Couette) | 14 300 s$^{-1}$ (cellule de Couette) |
| Taille moyenne | 5,0 $\mu$m | 5,2 $\mu$m | 5,8 $\mu$m |
| Largeur à mi-hauteur de la distribution | 5,5 $\mu$m | 5,8 $\mu$m | 6,8 $\mu$m |

**Exemple 3** : **Préparation de microcapsules solides grâce à une phase C3 peu visqueuse et différentes valeurs de cisaillement**

Cet exemple démontre que lorsqu'une composition C3 de viscosité inférieure à 2 000 mPa.s à 25°C est utilisée pour fabriquer des microcapsules, il est nécessaire de recourir à une étape supplémentaire de haut cisaillement afin d'obtenir des microcapsules monodisperses de taille inférieure à 20 $\mu$m.

*Composition de C1, C2 et C3 :*

- La composition de C1 et C2 est identique à celle de l'Exemple 1.

- La composition C3 est une solution d'alginate à 5% en masse, de viscosité 1 500 mPa.s.

*Fabrication des microcapsules :*

Les étapes a) et b) sont réalisées de façon identique à celles de l'Exemple 1.
Etape c) : L'émulsion (E2) obtenue est séparée en 3 fractions numérotées 1, 2, et 3 de volume égal qui sont soumises aux conditions de cisaillement suivantes :

| | Conditions de cisaillement |
|---|---|
| Fraction 1 | Pâle défloculeuse, cisaillement < 500 s$^{-1}$ |
| Fraction 2 | Cellule de Couette, cisaillement 6 300 s$^{-1}$ |
| Fraction 3 | Cellule de Couette, cisaillement 14 300 s$^{-1}$ |

Etape d) : Les fractions 1, 2, et 3 sont irradiées pendant 10 minutes à l'aide d'une source de lumière UV (Dymax LightBox ECE 2000) ayant une intensité lumineuse maximale de 0,1 W/cm$^2$ à une longueur d'onde de 365 nm, pour permettre la réticulation des capsules.
La distribution de taille des capsules ainsi obtenues est mesurée par technique de diffusion de la lumière à l'aide d'un Mastersizer 3000 (Malvern Instruments) équipé d'une cellule de meure Hydro SV. La taille moyenne des capsules et la largeur à mi-hauteur de la distribution de taille obtenues sont récapitulées dans le tableau ci-dessous. On voit clairement qu'il est nécessaire d'appliquer un cisaillement d'au moins 6 300 s$^{-1}$ afin d'obtenir des capsules monodisperses de taille inférieure à 20 $\mu$m.

| | Taille moyenne | Largeur à mi-hauteur de la distribution |
|---|---|---|
| Fraction 1 | 26 $\mu$m | 31 $\mu$m |
| Fraction 2 | 16 $\mu$m | 15 $\mu$m |
| Fraction 3 | 12,5 $\mu$m | 12 $\mu$m |

**Revendications**

**1.** Procédé de préparation de microcapsules solides comprenant les étapes suivantes :

a) l'addition sous agitation d'une composition C1, comprenant au moins un actif, dans une composition polymérique photoréticulable C2, les compositions C1 et C2 n'étant pas miscibles l'une dans l'autre,

ce par quoi on obtient une émulsion (E1) comprenant des gouttes de composition C1 dispersées dans la composition C2 ;

b) l'addition sous agitation de l'émulsion (E1) dans une composition C3, les compositions C2 et C3 n'étant pas miscibles l'une dans l'autre,

la viscosité de la composition C3 étant supérieure à la viscosité de l'émulsion (E1), et étant supérieure à 2 000 mPa.s à 25°C,

ce par quoi on obtient une émulsion double (E2) comprenant des gouttes dispersées dans la composition C3 ;

c) l'application d'un cisaillement à l'émulsion (E2), ladite vitesse de cisaillement appliquée étant inférieure à 1 000 s$^{-1}$,

ce par quoi on obtient une émulsion double (E3) comprenant des gouttes de taille contrôlée dispersées dans la composition C3 ; et

d) la photopolymérisation de la composition C2, ce par quoi on obtient des microcapsules solides dispersées dans la composition C3.

2. Procédé selon la revendication 1, dans lequel la composition C2 est un liquide dont la viscosité à 25°C est comprise entre 500 mPa.s et 100 000 mPa.s.

3. Procédé selon l'une quelconque des revendications 1 ou 2, dans lequel la composition C2 comprend au moins un monomère ou polymère, au moins un agent réticulant et au moins un photoinitiateur.

4. Procédé selon la revendication 3, dans lequel la composition C2 comprend de 0,001% à 70% en poids d'agent réticulant par rapport au poids total de composition C2.

5. Procédé selon l'une quelconque des revendications 1 à 4, dans lequel l'actif est solubilisé dans la composition C1 ou est dispersé sous la forme de particules solides dans la composition C1.

6. Procédé selon l'une quelconque des revendications 1 à 5, dans lequel la composition C3 comprend au moins un polymère branché, de préférence de poids moléculaire supérieur à 5 000 g.mol$^{-1}$, et/ou au moins un polymère de poids moléculaire supérieur à 5 000 g.mol$^{-1}$, et/ou des particules solides telles que des silicates.

7. Procédé selon l'une quelconque des revendications 1 à 6, dans lequel la viscosité de la composition C3 à 25°C est comprise entre 1 000 mPa.s et 100 000 mPa.s.

8. Procédé selon l'une quelconque des revendications 1 à 7, dans lequel la vitesse de cisaillement appliquée à l'étape c) est comprise entre 10 s$^{-1}$ et 1 000 s$^{-1}$.

9. Procédé selon l'une quelconque des revendications 1 à 8, dans lequel l'étape d) consiste à exposer l'émulsion (E3) à une source de lumière apte à initier la photopolymérisation de la composition C2.

10. Procédé selon la revendication 9, dans lequel la source de lumière est une source de lumière UV.

**Patentansprüche**

1. Verfahren zum Herstellen von festen Mikrokapseln, welches die folgenden Schritte aufweist:

a) Hinzufügen von einer Zusammensetzung C1, welche mindestens einen Aktivstoff aufweist, unter Rühren zu einer photovernetzbaren Polymerzusammensetzung C2, wobei die Zusammensetzungen C1 und C2 nicht miteinander mischbar sind,

wodurch man eine Emulsion (E1) erhält, welche Tropfen der Zusammensetzung C1 aufweist, welche in der Zusammensetzung C2 dispergiert sind,

b) Hinzufügen von der Emulsion (E1) zu einer Zusammensetzung C3 unter Rühren, wobei die Zusammensetzungen C2 und C3 nicht miteinander mischbar sind,

wobei die Viskosität der Zusammensetzung C3 größer ist als die Viskosität der Emulsion (E1) und größer ist als 2000 mPa.s bei 25°C,

wodurch man eine Doppelemulsion (E2) erhält, welche Tropfen aufweist, welche in der Zusammensetzung C3 dispergiert sind,

c) Anwenden einer Scherung auf die Emulsion (E2), wobei die besagte angewendete Schergeschwindigkeit kleiner als 1000 s$^{-1}$ ist,

wodurch man eine Doppelemulsion (E3) erhält, welche Tropfen mit kontrollierter Größe aufweist, welche in der Zusammensetzung C3 dispergiert sind, und

d) das Photopolymerisieren der Zusammensetzung C2, wodurch man feste Mikrokapseln erhält, welche in der Zusammensetzung C3 dispergiert sind.

**2.** Verfahren gemäß Anspruch 1, wobei die Zusammensetzung C2 eine Flüssigkeit ist, deren Viskosität bei 25°C zwischen 500 mPa.s und 100000 mPa.s liegt.

**3.** Verfahren gemäß irgendeinem der Ansprüche 1 oder 2, wobei die Zusammensetzung C2 zumindest ein Monomer oder ein Polymer, zumindest ein Vernetzungsmittel und zumindest einen Photoinitiator aufweist.

**4.** Verfahren gemäß Anspruch 3, wobei die Zusammensetzung C2 0,001% bis 70%, bezogen auf das Gewicht, Vernetzungsmittel im Verhältnis zum Gesamtgewicht der Zusammensetzung C2 aufweist.

**5.** Verfahren gemäß irgendeinem der Ansprüche 1 bis 4, wobei der Aktivstoff in der Zusammensetzung C1 gelöst ist oder in Form von festen Partikeln in der Zusammensetzung C1 dispergiert ist.

**6.** Verfahren gemäß irgendeinem der Ansprüche 1 bis 5, wobei die Zusammensetzung C3 aufweist zumindest ein verzweigtes Polymer, vorzugsweise mit einem Molekulargewicht, welches größer als 5000 g.mol$^{-1}$ ist, und/oder zumindest ein Polymer mit einem Molekulargewicht, welches größer als 5000 g.mol$^{-1}$ ist, und/oder feste Partikel, wie zum Beispiel Silikate.

**7.** Verfahren gemäß irgendeinem der Ansprüche 1 bis 6, wobei die Viskosität der Zusammensetzung C3 bei 25°C zwischen 1000 mPa.s und 100000 mPa.s liegt.

**8.** Verfahren gemäß irgendeinem der Ansprüche 1 bis 7, wobei die Schergeschwindigkeit, welche im Schritt c) angewendet wird, zwischen 10 s$^{-1}$ und 1000 s$^{-1}$ liegt.

**9.** Verfahren gemäß irgendeinem der Ansprüche 1 bis 8, wobei der Schritt d) daraus besteht, dass die Emulsion (E3) einer Lichtquelle ausgesetzt wird, welche geeignet ist, um das Photopolymerisieren der Zusammensetzung C2 zu initiieren.

**10.** Verfahren gemäß Anspruch 9, wobei die Lichtquelle eine UV-LichtQuelle ist.

**Claims**

**1.** Method for preparing solid microcapsules comprising the steps of:

a) the addition, with stirring, of a composition C1, comprising at least one active ingredient, in a photocrosslinkable polymer composition C2, the compositions C1 and C2 not being miscible with each other,

wherein an emulsion (E1) comprising drops of composition C1 dispersed in composition C2, is obtained;

b) the addition, with stirring, of the emulsion (E1) in a composition C3, the compositions C2 and C3 not being miscible with each other,

the viscosity of the composition C3 being greater than the viscosity of the emulsion (E1), and being greater than 2,000 mPa.s at 25°C,

wherein a double emulsion (E2) comprising drops dispersed in the composition C3, is obtained;

c) applying shear to the emulsion (E2), the applied shear rate being less than 1000 s$^{-1}$,

wherein a double emulsion (E3) is obtained comprising controlled-size drops dispersed in the composition C3; and

d) the photopolymerization of the composition C2, wherein solid microcapsules dispersed in the composition C3 are obtained.

**2.** Method according to claim 1, wherein the composition C2 is a liquid the viscosity of which at 25°C is between 500 mPa.s and 100 000 mPa.s.

**3.** Method according to any one of claims 1 or 2, wherein the composition C2 comprises at least one monomer or polymer, at least one crosslinking agent, and at least one photoinitiator.

**4.** Method according to claim 3, wherein the composition C2 comprises from 0.001% to 70% by weight of crosslinking agent relative to the total weight of composition C2.

**5.** Method according to any one of claims 1 to 4, wherein the active ingredient is solubilized in the composition C1, or is dispersed in the form of solid particles in the composition C1.

**6.** Method according to any one of claims 1 to 5, in which the composition C3 comprises at least one branched polymer, preferably with a molecular weight greater than 5000 $g.mol^{-1}$, and/or at least one polymer of molecular weight greater than 5,000 $g.mol^{-1}$, and/or solid particles such as silicates.

**7.** Method according to any of claims 1 to 6, wherein the viscosity of composition C3 at 25°C is between 1000 mPa.s and 100,000 mPa.s.

**8.** Method according to any one of claims 1 to 7, wherein the shear rate applied in step c) is between 10 $s^{-1}$ and 1000 $s^{-1}$.

**9.** Method according to any one of claims 1 to 8, wherein step d) comprises exposing the emulsion (E3) to a light source capable of initiating the photopolymerization of the composition C2.

**10.** Method according to claim 9, wherein the light source is a UV light source.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6335315 B

- US 5877145 A

**Littérature non-brevet citée dans la description**

- **FARID et al.** A review on phase change energy storage: materials and applications. *Energy Conversion and Management,* 2004, vol. 45 (9-10), 1597-1615
- **G. LI BASSI.** Les photoinitiateurs dans la réticulation des revêtements. *Double Liaison - Chimie des Peintures,* Novembre 1985, 34-41
- **HENRI STRUB.** Applications industrielles de la polymérisation photoinduite. *L'Actualité Chimique,* Février 2000, 5-13

- **MARC, J.M. ABADIE.** Photopolymères : considérations théoriques et réaction de prise. *Double Liaison - Chimie des Peintures,* 1992, 28-34
- **LIU et al.** *Polymer Chemistry,* 2013, vol. 4, 3431-3443
- **METZNER AB ; OTTO RE.** Agitation of non-Newtonian fluids. *AIChE J,* 1957, vol. 3, 3-10
- **WU, J et al.** Estimation of agitator flow shear rate. *AIChE J,* 2006, vol. 52, 2323-2332